# EUROPEAN PATENT APPLICATION

(11) **EP 4 787 384 A1**
(43) Date of publication of application: **05.08.2026**
(21) Application number: 25155356.6
(22) Date of filing: 31.01.2025
(51) Int. Cl.: G16H 50/20

(54) **SYSTEM AND METHOD FOR ASSISTING MULTIDISCIPLINARY DECISION-MAKING FOR TREATMENT RECOMMENDATIONS**

(71) Applicant: Universitätsmedizin der Johannes Gutenberg-Universität Mainz, 55131 Mainz (DE); Deutsches Forschungszentrum für Künstliche Intelligenz GmbH (DFKI), 67663 Kaiserslautern (DE)
(72) Inventor: Haferkamp, Axel, 55128 Mainz (DE); Höfner, Thomas, 4202 Eben (AT); Duwe, Gregor, 55130 Mainz (DE); Kauth, Verena, 55276 Oppenheim (DE); Mönch, Kerstin, 67663 Kaiserslautern (DE); Dengel, Andreas, 67697 Otterberg (DE); Mercier, Dominique, 66882 Hütschenhausen (DE); Rajashekar, Vikas, 67655 Kaiserslautern (DE); Junker, Markus, 66903 Gries (DE)
(74) Representative: Valvoda, Jakob

(57) **Abstract**

A system and a method for assisting multidisciplinary decision-making for treatment recommendations are described. A two-tier inference system with a first-tier trained model and at least one second-tier trained model is provided. The method includes accessing patient features derived from a clinical record of a patient, performing a high-level classification with the first-tier trained model using the patient features to identify a treatment category for the patient, performing a low-level classification with the at least one second-tier trained model using the patient features and the identified treatment category to identify one or more treatment options for the patient within the identified treatment category, determining for at least some of the patient features contribution scores, each contribution score indicating an impact of a corresponding patient feature on the identified treatment category or the identified one or more treatment options, and rendering at least one of the identified treatment category, the identified one or more treatment options, and one or more indications of the contribution scores on an interactive dashboard.

## Description

### FIELD OF DISCLOSURE

The present disclosure relates to a system and a method for assisting multidisciplinary decision-making for treatment recommendations, which may be based on patient data and clinical evidence. Specifically, the present disclosure relates to a two-tier inference system utilizing trained models to classify patient features and provide treatment options, with results rendered on an interactive dashboard.

### BACKGROUND

In the field of medical decision-making, particularly in the context of treatment recommendations, it is common to rely on multidisciplinary teams to evaluate patient data and clinical evidence. These teams, often referred to as "tumor boards" in oncology, consist of specialists from various medical disciplines who collaborate to determine the most appropriate treatment plans for patients. Known systems typically involve manual review and discussion of patient records, clinical trial data, and medical guidelines to reach a consensus on treatment options. This process can be time-consuming, prone to human error, and may lack consistency due to the subjective nature of individual expert opinions as well as risk of missing medical knowledge of latest guideline and trial evidence.

According to known approaches, the integration of patient data and clinical evidence into decision-making is often fragmented and lacks a standardized methodology. Traditional methods may involve the use of electronic health records (EHRs) and clinical decision support systems (CDSS), which provide some level of assistance by aggregating patient information and suggesting potential treatments based on predefined rules or algorithms. However, these systems often fall short in handling the complexity and variability of patient data, especially when dealing with unstructured data such as clinical notes or imaging reports. Furthermore, the ability to explain the rationale behind treatment recommendations is limited, making it challenging for clinicians to understand and trust the outputs of these systems.

Despite the substantial advances in the field of medical informatics, there remains a need for more sophisticated and reliable systems that can assist multidisciplinary teams in making informed treatment decisions, for example, in clinical oncology and other areas that require complex decision processes. Current systems often lack the capability to dynamically update recommendations based on new patient data or evolving clinical evidence. Additionally, the integration of clinical trial data into treatment recommendations is frequently inadequate, leading to missed opportunities for identifying the most effective treatments based on the latest research. The need for explainability and transparency in decision-making is also a critical aspect that is not sufficiently addressed by existing systems, which can hinder the adoption and trust of automated decision support tools among healthcare professionals.

### SUMMARY OF THE DISCLOSURE

It is therefore an object of the invention to provide a method and system for assisting multidisciplinary decision-making for treatment recommendations based on patient data and clinical evidence that at least partially overcomes the disadvantages of known systems.

A first aspect of the invention provides a method for assisting multidisciplinary decision-making for treatment recommendations. This is preferably based on patient data and/or clinical evidence. Preferably, the clinical evidence may address one or more of expert guidelines, corresponding clinical trials, an/or any other clinical evidence. The method comprises providing a two-tier inference system, including a first-tier trained model and at least one second-tier trained model, accessing patient features derived from a clinical record of a patient, performing a high-level classification with the first-tier trained model using the patient features to identify a treatment category for the patient, performing a low-level classification with the at least one second-tier trained model using the patient features and the identified treatment category to identify one or more treatment options for the patient within the identified treatment category, determining for at least some of the patient features contribution scores, each contribution score indicating an impact of a corresponding patient feature on the identified treatment category or the identified one or more treatment options, and rendering at least one of the identified treatment category, the identified one or more treatment options, and one or more indications of the contribution scores on an interactive dashboard.

The method may be a computer-implemented method. The method may be implemented in a computing device or on a system with one or more computing devices, including one or more processors or processing units (PUs) that may be coupled to each other or communicate with each other, and that may be configured to perform individual method steps. The one or more processors or PUs may implement hardware and/or software modules that execute the individual method steps. Examples may include at least one inference PU or engine configured to perform the high-level or low-level classifications; a scoring PU; and/or a rendering PU or renderer that may perform one or more steps of the method according to the first aspect or implementations of the first aspect.

The subject matter described is a method for aiding multidisciplinary decision-making in treatment recommendations using patient data and clinical evidence. It involves a two-tier inference system with a first-tier trained model and one or more second-tier trained models. Initially, patient features are accessed from the patient's clinical record. The first-tier trained model, which may also be referred to throughout the description as first trained model or first model, performs a high-level classification using these features to identify a treatment category. The one or more second-tier trained model(s), which may each also be referred to throughout this description as a second trained model or second model, may then performs a low-level classification using the features and the identified treatment category to determine one or more treatment options within the identified category. Contribution scores are determined for at least some of the patient features in both predictions, indicating the impact of each feature on the identified treatment category (predicted using the first-tier trained model) or treatment options (predicted using the one or more second-tier trained models). Finally, the identified treatment category, treatment options, and/or contribution scores, in any combination, are rendered on an interactive dashboard.

The two-tier inference system refers to a system with two levels of trained models, where the first-tier trained model provides a broad classification and the at least one second-tier trained model offers detailed recommendations. The first-tier trained model (or initial model) is used for high-level classification to identify a general treatment category. The at least one second-tier trained model (or subsequent model) is used for low-level classification to identify specific treatment options within the identified category. The term high-level classification can be used to designate an initial, rather general classification, specifically a broad categorization of treatment options based on the patient features. The term low-level classification can be used to designate a subsequent, rather detailed classification, specifically a detailed identification of specific treatment options within the high-level category, using the patient features. Accordingly, the high-level classification could also be referred to as the initial classification or simply the first classification. The low-level classification could also be referred to as the subsequent classification or simply the second classification.

The two-tier inference system may include one or more second-tier trained models. Each of the one or more second-tier trained models may be used during the low-level classification. Each second-tier trained model may be trained for particular treatment options, such as types of surgical procedures, (anti-cancer) drug medication, care procedures, radiation, or PSMA, and the like. Thus, each second-tier trained model used in the low-level classification may be considered as a best suited model for respective treatment options.

Accordingly, the initial classification by the first-tier model may indicate which therapy category is valid, for example, surgery or medication. In this case, for example, a second-tier model for surgery may be invoked to specify the surgical procedure, and a second-tier model for medication may be invoked to further define the medication treatment. It is also possible that the respective second-tier model identifies multiple treatment options within the identified category, such as multiple valid therapies (e.g., two different medications).

The patient features may represent characteristics or data points derived from the patient's clinical record, such as medical history, lab results, imaging data, pathological data, histological data and other relevant clinical information. The patient features could be dynamically updated with current results.

It is to be understood that the first-tier trained model and one or more of the second-tier trained models may operate on subsets of the patient features or all patient features. For example, a first subset of patient features may be derived from the clinical record of the patient and used to perform the high-level classification with the first-tier trained model. A second subset of patient features may be derived from the patient features. This may be based on the identified treatment category or any other results of the high-level classification. The second subset of patient features may be used to perform low-level classification with the second-tier trained model. As such, the method may include the step of accessing a first subset of patient features derived from the clinical record of the patient, and accessing a second subset of the patient features of the patient, wherein the second subset may be based on the identified treatment category. The first and the second subsets may overlap with each other or may be distinct subsets of the patient features. The first and the second subsets may also encompass all patient features or a part of the patient features, and, thus, may be identical. In one embodiment, the first-tier trained model and each one of the second-tier trained models may access all patient features. In this case, each first- and second-tier trained models may utilize those data in the subsets they have been trained on. Defining respective first and second subsets may be used to pre-select data required by the trained models.

The contribution scores may refer to numerical values indicating an impact of individual patient features on the classification and treatment recommendations generated by the high-level and the low-level classifications.

The interactive dashboard may represent a multi-modal user interface that may provide information in different modalities, such as visual data, audio data or haptic data, to name a few. The interactive dashboard may, for example, visualize the AI-generated treatment recommendations, contribution scores, and other relevant information in an interactive manner for easy interpretation by, for example, medical experts or professionals.

Advantages of the method include the ability to provide initial treatment predictions based on potentially limited set of patient data. This is used for a more detailed prediction, guided by the initially estimated treatment prediction and potentially using additional data of the clinical record that may be acquired at a later point in time. This approach allows for the use of independent machine learning models, trained for particular tasks, enhancing the accuracy and explainability of the treatment recommendations. The use of contribution scores helps in understanding the influence of specific patient features on the recommendations, thereby improving the transparency and reliability of the decision-making process. The interactive dashboard facilitates easy access to and interpretation of the recommendations, contributing to higher quality of clinical treatment.

In a first implementation of the method according to the first aspect, contribution scores are determined using an explanation engine configured to access the patient features in order to provide contribution scores for the patient. Preferably, the explanation engine may further provide global contribution scores across patient cohorts. The implementation utilizes an explanation engine as a specific mechanism for determining contribution scores. The explanation engine may be configured to access patient features, which are derived from the clinical record of the patient and are used in the high-level and low-level classifications respectively. The explanation engine's role is to provide contribution scores for the patient, which indicate the impact of each patient feature on the identified treatment category or the identified treatment options. Additionally, the explanation engine is capable of generating global contribution scores across patient cohorts, which means it can analyze and provide insights not only on an individual patient level but also on a broader scale, encompassing groups of patients with similar characteristics and/or with the same recommendation. This feature enhances the method by offering a more comprehensive understanding of how different patient features influence treatment decisions, both on a personal level and across a wider patient population. The inclusion of the explanation engine thus brings a layer of transparency and interpretability to the decision-making process, allowing healthcare providers to better understand the rationale behind the treatment recommendations. This can facilitate more informed and confident decision-making, as well as potentially uncover patterns and trends within patient cohorts that could inform future clinical practices and guidelines. The ability to provide both individual and global contribution scores ensures that the system is not only tailored to the specific needs of each patient but also contributes to the overall knowledge and improvement of treatment strategies in the medical field.

In a further implementation of the method according to the first aspect, the method further comprises generating labeled training data. The labeled training data may be based on clinical datasets, for example. The clinical datasets may include, for example, clinical records and/or previous decisions on treatment categories and/or treatment options based on patient features. For example, the clinical datasets may include previous tumor board decisions and/or any other data designating previous decisions, classifications or inferences on treatment categories and/or treatment options. The implementation introduces the additional step of generating the labeled training data and subsequently using the labeled training data to train the first-tier trained model and/or the at least one second-tier trained model and/or both. For example, the labeled training data may be generated by separating previous tumor board decisions, clinical datasets or clinical records with multiple treatment options into separate cases, wherein each case defines one of the treatment options. A recommendation of multiple treatment options may be handled later on by the (first and second) trained models. This enables assisting multidisciplinary decision-making for treatment recommendations by ensuring that the models are trained on data that is specifically labeled according to clinical records or datasets (that may include, for example, previous tumor board decisions), which improves the accuracy and relevance of the models' predictions. The generation of labeled training data involves the process of annotating clinical records with specific labels that are relevant to the treatment categories and options, thereby creating a robust dataset that can be used to train the models effectively. By training the first-tier trained model or the at least one second-tier trained model or both with labeled training data, the method ensures that the models are well-equipped to perform high-level and low-level classifications with greater precision. The high-level classification by the first-tier trained model identifies a treatment category for the patient using a first subset of patient features, while the low-level classification by the at least one second-tier trained model identifies one or more treatment options within the identified treatment category using a second subset of patient features and the identified treatment category. The inclusion of labeled training data directly impacts the performance of these models by providing them with a rich source of information that is directly derived from clinical records, thereby enhancing their ability to accurately determine treatment categories and options. Furthermore, the use of labeled training data ensures that the contribution scores, which indicate the impact of corresponding patient features on the identified treatment category or treatment options, are based on well-trained models, thus improving the reliability of the scores rendered on the interactive dashboard.

In a further implementation of the method according to the first aspect, datasets in the training data with multiple labels are identified and duplicated. The implementation introduces a mechanism for handling datasets within the training data that possess multiple labels. This mechanism involves identifying such datasets and then duplicating them, with each duplicated dataset containing (preferably) exactly one different label from the multiple labels originally associated with the dataset. This feature brings a significant enhancement to the method described in the aspect by addressing the complexity and variability inherent in datasets with multiple labels. In the context of the two-tier inference system, which includes a first-tier trained model and at least one second-tier trained model, the introduction of this feature ensures that the training data is more robust and comprehensive. By duplicating datasets with multiple labels, the method can effectively increase the diversity and richness of the training data, allowing the trained models to better generalize and make more accurate predictions. This is particularly important in a multidisciplinary decision-making process for treatment recommendations, where patient data and clinical evidence can be multifaceted and complex. The duplication of datasets with different labels ensures that the models are exposed to a wider range of scenarios and variations, thereby enhancing their ability to classify and recommend treatment options with greater precision. Moreover, this feature contributes to the reliability of the contribution scores determined for patient features in the first and second subsets. By ensuring that the training data reflects a comprehensive range of labels, the method can more accurately assess the impact of each patient feature on the identified treatment category or treatment options.

The features in this context involve the identification and duplication of patients in the datasets, which helps preparing the training data for the models, in particular in scenarios with limited training datasets and ensures that the same patient and all its duplicated entries are only present in one dataset, such as training, validation, test. This process ensures that the models are trained on a dataset that is representative of the complexities encountered in real-world clinical scenarios, thus improving the overall effectiveness and accuracy of the method in assisting multidisciplinary decision-making for treatment recommendations.

In a further implementation of the method according to the first aspect, the patient features are matched to records in a database with clinical trial data using a third trained model. The implementation introduces a third trained model that facilitates the matching of patient features to records in a database containing clinical trial data. For example, the third trained model may be an already trained model, such as a Large Language Model (LLM) or any other suitable trained model. This matching process enables the identification of one or more records that specify treatment characteristics relevant to the identified treatment category and the one or more treatment options for the patient. This involves an interaction between the patient features, the third trained model, and the database with clinical trial data. The patient features, which are derived from the clinical record of a patient, are used as input for the third trained model. This model processes the patient features to perform a matching operation against the records in the clinical trial database. The result of this operation is the identification of records that contain treatment characteristics pertinent to the treatment category and options previously identified by the two-tier inference system comprising the first-tier trained model and the at least one second-tier trained model. The integration of the third trained model adds a layer of depth to the automated decision-making process by incorporating external clinical trial data, thereby enhancing the comprehensiveness and relevance of the treatment recommendations. The identified treatment characteristics are then rendered on the interactive dashboard, which serves as a user interface for displaying the results of the decision-making process. This rendering allows healthcare professionals to obtain not only the identified treatment category and options but also the associated treatment characteristics derived from clinical trial data. The inclusion of these characteristics provides additional context and evidence that can inform treatment decisions, thereby supporting a more informed and evidence-based approach to patient care. This feature underscores the importance of integrating diverse data sources and advanced modeling techniques in the development of sophisticated decision-support systems in healthcare.

In a further implementation of the method according to the first aspect, matching the patient features to records in the database comprises parsing, using the third trained model, the records including unstructured clinical trial data into structured trial criteria, comparing the patient features against the structured trial criteria, and identifying the treatment characteristics in the structured trial criteria matching the patient features. The implementation introduces specific mechanisms for matching patient features to records in a database. This involves parsing records that include unstructured clinical trial data into structured trial criteria. By converting unstructured data into a structured format, the system can more effectively compare the patient features against these structured trial criteria. This process allows for the identification of treatment characteristics within the structured trial criteria that match the patient features. The mechanism ensures that the unstructured clinical trial data is transformed into a structured format, making it easier to compare and match with patient features. The comparison of patient features against the structured trial criteria facilitates a more accurate identification of relevant treatment characteristics, thereby improving the precision of treatment recommendations.

In a further implementation of the method according to the first aspect, the treatment characteristics include at least one of eligibility status and survival data. The implementation enriches the decision-making process by incorporating critical treatment characteristics that are pivotal in evaluating and recommending treatment options. This context involves the interaction between the patient data, the trained models, and the treatment characteristics. The first-tier trained model performs a high-level classification using a subset of patient features to identify a treatment category. This classification is then refined by the at least one second-tier trained model, which uses the patient features and the identified treatment category to pinpoint specific treatment options. Eligibility status could refer to the patient's suitability for a particular treatment based on medical criteria, while survival data might provide statistical insights into the expected outcomes of the treatment options. These characteristics could be used as additional data points that influence the model's decision-making process. The features ensure that the treatment recommendations are not only based on patient features and clinical evidence but also on critical treatment characteristics that can significantly impact the decision-making process. By incorporating eligibility status and survival data, the method can provide more personalized and potentially more effective treatment recommendations. The rendering of the identified treatment category, treatment options, and contribution scores on an interactive dashboard, as described in the aspect, is further enriched by the inclusion of treatment characteristics, providing healthcare professionals with a comprehensive view of the factors influencing the treatment recommendations. This holistic approach facilitates informed decision-making and enhances the overall effectiveness of the automated treatment recommendation process.

In a further implementation of the method according to the first aspect, the high-level classification identifies the treatment categories, including at least one of surgery, anti-cancer drugs, follow-up care, and/or best supportive care, to name a few, in any combination. Accordingly, the identified treatment categories can include at least one of surgery, anti-cancer drugs, follow-up care, and best supportive care. Further treatment categories could be used to characterize the treatment in the initial step and are encompassed by the disclosure. By including categories such as surgery, anti-cancer drugs, follow-up care, and best supportive care, the method is equipped to handle a wide range of medical scenarios, thereby increasing its applicability and utility in real-world clinical settings. This specificity also aids in the determination of contribution scores for patient features, as it provides a clear context within which the impact of each feature can be assessed.

In a further implementation of the method according to the first aspect, the low-level classification identifies the one or more treatment options, including at least one of types of surgical procedures, at least one anti-cancer drug, and/or at least one care procedure, to name a few, in any combination. By explicitly identifying types of surgical procedures, anti-cancer drugs, and care procedures, the method ensures that the treatment options provided are not only relevant to the patient's condition but also tailored to the specific needs and circumstances of the patient. It is to be understood that further treatment options can be defined and are encompassed by the disclosure. Defining treatment options allows for a more targeted approach to treatment planning, potentially improving patient outcomes by providing more precise and actionable recommendations. This feature implies that the at least one second-tier trained model is equipped with the necessary knowledge and data to distinguish between different types of surgical procedures, anti-cancer drugs, and care procedures, suggesting a robust and well-trained system capable of making nuanced and informed decisions.

In a further implementation of the method according to the first aspect, the method further comprises extracting patient features from the clinical records and preprocessing the patient features. This may include including one or more of normalizing and standardizing numerical data, encoding categorical variables, and parsing complex data types into structured features. The implementation emphasizes the importance of transforming raw patient data into a format that is suitable for analysis by the inference system. This context involves the extraction of patient features from clinical records, which ensures that the data is in a usable form for subsequent processing. The preprocessing of patient features is a critical step that may include one or more specific tasks, in any combination: normalizing and standardizing numerical data, encoding categorical variables, and parsing complex data types into structured features. Further preprocessing steps could be added. An optional normalizing and standardizing of numerical data may ensure that the data is on a consistent scale, which is essential for accurate analysis and comparison. Encoding categorical variables involves converting categorical data into a numerical format that can be easily processed by the trained models. Parsing complex data types into structured features involves breaking down intricate data formats into simpler, more manageable components that can be effectively utilized by the inference system. By ensuring that the data is in a structured and standardized format, the preprocessing step enhances the accuracy and reliability of the high-level and low-level classifications performed by the trained models. The preprocessing of patient features contributes to the determination of contribution scores, which indicate the impact of specific patient features on the identified treatment category or treatment options.

In a further implementation of the method according to the first aspect, the patient features include one or more of demographic data, such as age, gender; clinical data, such as cancer type, tumor stage, tumor grade, comorbidities, prior treatments and treatment history; biomarker data, such as PD-L1 expression, genetic mutations; laboratory results, such as renal function, liver function, blood counts; histopathological findings, symptomatology, such as pain levels, weight loss; radiological reports that may relate on (or include) imaging data, such as CT scans, MRI scans, PET scans, and tumor measurements; and parsed complex data types, such as scores for programmed death-ligand 1 or other biomarkers. The patient features may encompass a wide range of data types, including demographic data such as age and gender, clinical data which covers aspects like cancer type, tumor stage, tumor grade, comorbidities, prior treatments, and treatment history, in any combination. Further patient features are encompassed by the disclosure. Additionally, biomarker data such as PD-L1 expression and genetic mutations are included, as well as laboratory results that might involve renal function, liver function, and blood counts. Histopathological findings, symptomatology such as pain levels and weight loss, and. Imaging data, which includes CT scans, MRI scans, PET scans, and tumor measurements, along with parsed complex data types like scores for programmed death-ligand 1 or other biomarkers, and corresponding (radiological) reports may be part of the patient features. The inclusion of diverse types of patient features enhances the method by providing a comprehensive dataset that can be used to perform high-level and low-level classifications with the trained models. This comprehensive dataset allows for a more accurate and personalized identification of treatment categories and treatment options for the patient.

In a further implementation of the method according to the first aspect, the first-tier trained model and the at least one second-tier trained model operate on the patient features. This may include using (radiological) reports that rely on (or include) image data or pathological parameters as input for the first-tier trained model or the at least one second-tier trained model. However, it is to be understood that other patient features can be used as input (or as training data) for the trained models.

In a further implementation of the method according to the first aspect, the first-tier trained model and the at least one second-tier trained model include one or more of neural networks, support vector machines or Boosting Algorithms, Tree-based Approaches or ensemble methods. Preferably, each trained model may be trained on labeled datasets to perform supervised learning for high-level classification of treatment categories and low-level classification of treatment options. The labeled datasets may be clinical datasets, which may include previous tumor board decisions, for example. The first-tier trained model and the at least one second-tier trained model can include various modules known from machine learning approaches, including neural networks, support vector machines, or ensemble methods. Neural networks, known for their ability to model complex patterns and relationships in data, can be particularly effective in handling the diverse and intricate nature of patient data and clinical evidence. Support vector machines, on the other hand, are robust classifiers that can be used for both linear and non-linear classification tasks, making them suitable for distinguishing between different treatment categories and options. Ensemble methods, which combine multiple learning algorithms to obtain better predictive performance than could be obtained from any of the constituent learning algorithms alone, offer a way to enhance the accuracy and reliability of the classifications. The implementation further specifies that each trained model is preferably trained on labeled clinical datasets to perform supervised learning. This aspect underscores the importance of using real-world clinical data to train the models, ensuring that the classifications are grounded in actual clinical scenarios and evidence. Supervised learning, which involves training a model on a labeled dataset, allows the model to learn the mapping from input features to the desired output, thereby enabling it to make informed predictions about treatment categories and options. This training approach enhances the model's ability to accurately classify treatment categories at a high level and treatment options at a low level, based on the patient features accessed from the clinical record. By specifying the use of labeled (clinical) datasets, the implementation emphasizes the necessity of using high-quality, annotated data to achieve reliable and clinically relevant outcomes.

In a further implementation of the method according to the first aspect, the third trained model is a large language model. The aspect outlines a method that involves providing a two-tier inference system with a first-tier and at least one second-tier trained model, accessing patient features, performing classifications to identify treatment categories and options, determining contribution scores, and rendering results on an interactive dashboard. The introduction of a large language model as a third (already) trained model defines a sophisticated mechanism for processing and interpreting complex patient data and clinical evidence. Large language models are known for their ability to understand and generate human-like text, which implies that this model could facilitate more nuanced and context-aware interpretations of clinical evidence, and enable mapping of patient features on the (unstructured) clinical evidence. Overall, the inclusion of a large language model as the third already trained model in the method enhances the system's capability to process, interpret, and communicate complex patient data mapped on clinical evidence, thereby improving the quality and effectiveness of the treatment recommendations provided to healthcare professionals.

In a further implementation of the method according to the first aspect, the two-tier inference system is further enhanced by preprocessing techniques for structured feature extraction, explainability frameworks for contribution scoring, and large language models for matching clinical trial data against patient records. The preprocessing techniques for structured feature representation serve as a mechanism to refine and organize the patient data before it is used by the inference models. This ensures that the data fed into the models is clean, relevant, and in a format that maximizes the models' performance. By structuring the patient features from the patient record effectively, the system can more accurately identify treatment categories and options, thereby enhancing decision-making. The inclusion of explainability frameworks for contribution scoring adds another layer by providing transparency and interpretability to the model's predictions. These frameworks allow for the determination of contribution scores, which indicate the impact of specific patient features on the treatment recommendations. This not only aids healthcare professionals in understanding the rationale behind the model's suggestions but also facilitates trust and confidence in the system's outputs. Moreover, the use of large language models for matching clinical trial data against patient data introduces a sophisticated mechanism for handling complex and varied data sources. Clinical trial data is often unstructured and challenging to integrate into decision-making processes. By employing large language models, the system can effectively match this data against patient data formats that are compatible with the inference models, thereby enriching the dataset and potentially uncovering new insights for treatment recommendations. This feature significantly expands the system's capability to leverage a broader range of clinical evidence, ultimately leading to more informed and comprehensive treatment decisions.

In a further implementation of the method according to the first aspect, the first-tier trained model uses supervised learning to perform high-level classification of patient features into treatment categories. Preferably, one or more of the at least one second-tier trained model utilize context-aware supervised learning to refine the treatment category into a corresponding one or more treatment options based on patient features. The first-tier trained model utilizes supervised learning to perform high-level classification of general patient features into broad treatment categories. This implies that the model has been trained on a labeled dataset where the outcomes are known, allowing it to learn the mapping from input features to output categories effectively. The use of supervised learning in the first model ensures that the classification process is guided by pre-existing knowledge and patterns derived from historical data, thereby enhancing the accuracy and reliability of the treatment category identification. Each of the one or more second-tier trained models may employ context-aware supervised learning to refine the treatment category into a respective specific treatment option based on detailed patient features. Context-aware supervised learning implies that the model not only considers the input features but also incorporates contextual information relevant to the patient's specific circumstances. Thus, each of the second-tier trained models may utilizes context-aware supervised learning to refine the treatment category into a specific treatment options based on detailed patient features. By refining the treatment category into specific options, the second model enhances the granularity of the recommendations, which can lead to more effective and personalized patient care.

In a further implementation of the method according to the first aspect, the first-tier trained model is trained on labeled data representing treatment categories, and operates on patient features, to determine a most appropriate treatment category. Preferably, the patient features may include including one or more of age, cancer type, tumor stage, comorbidities, and prior treatments. The first-tier trained model is trained on labeled data that represents treatment categories, which implies a supervised learning approach where the model learns to associate specific patient features with predefined treatment categories. The patient features that the model operates on include, for example, age, cancer type, tumor stage, comorbidities, and prior treatments. These features provide the model with a comprehensive view of the patient's clinical profile, allowing it to make informed decisions regarding the most appropriate treatment category.

In a further implementation of the method according to the first aspect, the at least one second-tier trained model is trained on labeled data, which may be specific to treatment categories, and uses the identified treatment category as an input parameter to focus the low-level classification and refine the one or more treatment options. The second-tier trained model may access all patient data. The at least one second-tier trained model is characterized by its training on labeled data that is specific to treatment categories. This implies a targeted approach where the model is refined and specialized based on the treatment categories it is intended to address. The identified treatment category serves as an input parameter to decide for the corresponding second-tier trained model, which focuses the low-level classification process. The input parameter acts as a communication link between the high-level classification performed by the first-tier trained model and the low-level classification by the at least one second-tier trained model. By using the identified treatment category as an input to decide for the corresponding second-tier trained model, the second trained model is able to refine the one or more treatment options available to the patient. The incorporation of all patient features further enriches the data set used for classification, as the model can select on its own which features are relevant, contributing to a more comprehensive analysis and ultimately leading to better-informed treatment decisions.

In a further implementation of the method according to the first aspect, the interactive dashboard is provided as a graphical user interface, including one or more dynamic components for interactive visualizations. Preferably, the graphical user interface may be implemented using a web-based technology framework, such as one or more of HTML5, CSS, Python, and JavaScript. The implementation facilitates user interaction with the system through a (web-based) interface, which is accessible across various devices and platforms. Various technologies can be used to define the functional GUI across various devices and platforms. This may include frameworks or modules of an operating system and/or web-based techniques. For example, by employing HTML5, CSS, Python, and JavaScript, the system may ensure a responsive and dynamic user experience, allowing for seamless interaction with the treatment recommendations and contribution scores rendered on the dashboard. HTML5 may provide the structural foundation for the GLTI, enabling the incorporation of multimedia elements and advanced graphics without the need for additional plugins. CSS may be utilized to style the interface, ensuring that the visual presentation is functionally effective. JavaScript and Python may add interactivity to the GUI, allowing for real-time updates and dynamic content changes based on user inputs or system outputs. The implementation specifies an inclusion of one or more dynamic components for interactive visualizations. Interactive visualizations can include charts, graphs, and other graphical representations that allow users to explore data by interacting with the visual elements. This interactivity enables users to gain insights into the treatment recommendations and contribution scores, and to influence the automated inference and decision-making by manipulating the visualizations, such as zooming in on specific data points or filtering data sets to focus on or highlight particular aspects of the patient information. The dynamic nature of these components ensures that the visualizations are updated in real-time as new data is accessed or as the user interacts with the system. This real-time interactivity is advantageous for multidisciplinary decision-making, as it allows healthcare professionals to make informed decisions based on the most current and relevant data available. Overall, the implementation enhances the aspect by specifying the use of a web-based technology framework for the GLTI and the inclusion of dynamic components for interactive visualizations, thereby improving the accessibility, functionality, and user experience of the interactive dashboard.

In a further implementation of the method according to the first aspect, the method further comprises filtering of patient features in real-time to dynamically update the contribution scores and treatment options. The filtering may be based on declaring individual patient features as not set, thus skipping processing of the individual patient features. Real-time filtering allows for a continuous and immediate processing of patient data as it becomes available, ensuring that the contribution scores and treatment options are always based on the most current and relevant information. "Real-time" within the meaning of this disclosure defines a direct processing of available (new) data without further delay. Real-time processing of new data could be, therefore, understood in contrast to, for example, a planned processing at a particular (delayed) point of time, such as on a particular day of a week or at a particular time. This dynamic updating enhances the responsiveness and accuracy of the decision-making process, as it can adapt to new patient data without delay. By incorporating real-time filtering, the system can provide more precise and timely treatment recommendations, which may be beneficial in a clinical setting where patient conditions can change rapidly. This feature ensures that the treatment recommendations are not only based on historical data but are also continuously refined based on the latest patient information. This leads to a more personalized and effective treatment plan for the patient, as the system can quickly adjust to any changes in the patient's condition. Additionally, the real-time aspect ensures that the healthcare providers have access to the most up-to-date information, which can aid in making more informed and accurate decisions. Overall, the introduction of real-time filtering and dynamic updating of contribution scores and treatment options significantly enhances the method's ability to provide timely, accurate, and personalized treatment recommendations, thereby improving patient outcomes and the efficiency of the clinical decision-making process.

In a further implementation of the method according to the first aspect, the interactive dashboard further provides evidence-based annotations linking treatment options to clinical trial data or medical guidelines, and interactive links to a source of clinical trial data for validation. The annotations serve to link the identified treatment options to clinical trial data or medical guidelines, thereby providing a robust framework for validating the treatment recommendations. The feature integrates external clinical evidence directly into the interactive dashboard, allowing healthcare professionals to make more informed decisions. The interactive links to a source of clinical trial data for validation further offer users direct access to the underlying evidence that supports the treatment options. The interactive dashboard becomes a more comprehensive tool for multidisciplinary decision-making, as it not only presents treatment options but also contextualizes them within the broader landscape of clinical evidence. This integration of evidence-based annotations and interactive links enhances the utility of the dashboard by bridging the gap between patient-specific data and general clinical knowledge, in an automated manner.

In a further implementation of the method according to the first aspect, access to the interactive dashboard is controlled via secure authentication. Access to the interactive dashboard, which is a key component for rendering treatment categories, options, and contribution scores, is controlled through secure authentication. This ensures that only authorized personnel can access sensitive patient information, thereby enhancing the security of the system. The secure authentication mechanism could involve various methods such as password protection, biometric verification, or multi-factor authentication. This feature maintains the confidentiality and integrity of patient data, especially in a healthcare setting where unauthorized access could lead to significant privacy breaches.

In a further implementation of the method according to the first aspect, the interactive dashboard is configured for cross-platform accessibility. Thereby, preferably, providing seamless synchronization of data across devices. The cross-platform accessibility implies that the dashboard can be accessed from different types of devices, such as computers, tablets, and smartphones, regardless of the operating system or platform they use. As an example, the operating system may include a Windows operating system, Linux, or Mac. This feature ensures that users can interact with the dashboard and view the treatment recommendations and contribution scores on any device they choose. Any updates or changes made to the data on one device may be automatically reflected on all other devices that access the dashboard. This synchronization can be achieved through cloud-based technologies or a centralized database that stores the patient data and treatment recommendations. The cross-platform accessibility and seamless synchronization also facilitate collaboration among healthcare professionals who may be using different devices (at different locations) to access the dashboard.

In a further implementation of the method according to the first aspect, the method further comprises dynamically updating, using a back-end service, rendered data on the interactive dashboard in real-time based on new user input or updated clinical trial datasets. The implementation introduces a mechanism for dynamically updating rendered data on an interactive dashboard in real-time, utilizing a back-end service. The dynamic updating capability is particularly relevant in the context of tumor board discussions, where new insights, observations, or decisions may emerge that necessitate immediate reflection in the treatment recommendations or options displayed. By incorporating real-time updates, the method ensures that the multidisciplinary team has access to the most accurate and relevant data, thereby supporting more informed decision-making. The real-time updates allow the dashboard to serve as a living document that evolves with the ongoing discussions and data inputs. This capability not only improves the accuracy of the information presented but also facilitates a more collaborative and dynamic decision-making process among the multidisciplinary team. The back-end service can directly render the data. Thus, the terminal devices may not require rendering capabilities, for example. This may further enhance cross-platform distribution of the interactive dashboard. Input on the terminal devices can be transferred back to the back-end service, in order to synchronize the input.

In a further implementation of the method according to the first aspect, the method is applicable in medical fields requiring multidisciplinary decision-making for treatment recommendations based on patient data and/or clinical evidence. This may preferably include oncology, cardiology, and neurology, specifically. Most preferably the method is applicable in urological, gastrointestinal, gynecological, or thoracic oncology.

As such, preferably, the method may be for assisting tumor board decision-making. Most preferably, the method is for assisting tumor board decision-making in urological oncology. The implementation introduces a specific application of the method for assisting multidisciplinary decision-making in the context of tumor board decision-making, with a preference for its use in urological oncology. In particular, the method may be advantageously applied for decision-making in the context of Urinary Tract Carcinoma (UC), Renal Cell Carcinoma (RCC), or Prostate Carcinoma, to name a few. However, the method is not limited to these specific entities and may also be extended to other oncological and non-oncological disciplines where similar multidisciplinary decision-making processes are required.

According to a second aspect of the disclosure, one or more computer-readable media store instructions thereon that, when executed by a computing device, configure the computing device to perform a method according to any implementation of the first aspect. For example, the computing device can be configured to provide a two-tier inference system, including a first-tier trained model and at least one second-tier trained model, access patient features derived from a clinical record of a patient, perform a high-level classification with the first-tier trained model using the patient features to identify a treatment category for the patient, perform a low-level classification with the at least one second-tier trained model using the patient features and the identified treatment category to identify one or more treatment options for the patient within the identified treatment category, determine for at least some of the patient features contribution scores, each contribution score indicating an impact of a corresponding patient feature on the identified treatment category or the identified one or more treatment options, and render at least one of the identified treatment category, the identified one or more treatment options, and one or more indications of the contribution scores on an interactive dashboard.

According to a third aspect, the disclosure provides a system for assisting multidisciplinary decision-making for treatment recommendations, wherein the system is configured to perform a method according to any implementation of the first aspect.

The functional configuration may include preprocessing patient data, performing high-level classification of treatment types, and providing detailed therapy recommendations. The system uses machine learning techniques, including traditional methods and novel neural networks, to deliver explainable support for treatment decisions.

For example, the system can be configured to provide a two-tier inference system, including a first-tier trained model and at least one second-tier trained model, access patient features derived from a clinical record of a patient, perform a high-level classification with the first-tier trained model using the patient features to identify a treatment category for the patient, perform a low-level classification with the at least one second-tier trained model using the patient features and the identified treatment category to identify one or more treatment options for the patient within the identified treatment category, determine for at least some of the patient features contribution scores, each contribution score indicating an impact of a corresponding patient feature on the identified treatment category or the identified one or more treatment options, and render at least one of the identified treatment category, the identified one or more treatment options, and one or more indications of the contribution scores on an interactive dashboard.

In this context, the term "system" may refer to a combination of hardware and software components that work together to achieve the desired functionality. "Multidisciplinary decision-making" indicates that the system is configured to support collaboration among experts from various medical fields to make informed treatment decisions. "Treatment recommendations" can be understood as suggestions provided by the system regarding the best course of action for patient care. "Patient data" may encompass all relevant information about the patient, including medical history, lab results, and other health records. "Clinical evidence" may refer to data from clinical studies and trials that support the treatment recommendations.

The system may provide quick initial treatment predictions based on initial patient data, followed by more precise recommendations, which may include further patient information once this is available. The two-stage approach allows for efficient and accurate decision-making. Additionally, the system's use of machine learning to handle complex data and generate multi-label predictions ensures that it can offer nuanced and tailored treatment recommendations. A preferred inclusion of clinical trial data further enhances the evidence-based nature of the recommendations.

In a first implementation of the system according to the third aspect, the system further comprises one or more computing devices and at least one database interconnected via a network, and at least one interactive dashboard.

The implementation incorporates one or more computing devices and at least one database, all interconnected via a network, along with at least one interactive dashboard. The inclusion of these components enhances the system's ability to facilitate communication and data exchange between various elements involved in the decision-making process. The computing devices serve as the primary processing units that execute the system's algorithms and manage data flow. For example, the computing devices may execute the processing steps of implementations of the first aspect. The database acts as a centralized repository for storing patient data and clinical evidence. By being interconnected via a network, the computing devices and the database can communicate seamlessly, allowing for real-time data updates and retrieval. This ensures that the system can access the most current information. The interactive dashboard is a user interface component that provides a visual representation of the data and insights generated by the system. It allows users to interact with the data. The dashboard can display various metrics, trends, and recommendations.

It is to be understood that processing steps of implementations of the method according to the first aspect may be included as functional components of a further implementation of the third aspect, in any combination. The functional components of the system may be configured to perform the respective processing step(s). Moreover, system features according to an implementation of the third aspect may be included as processing steps of a further implementation of the first aspect, in any combination. The processing steps may define the functionality of the system features.

### BRIEF DESCRIPTION OF THE DRAWINGS

To illustrate the technical features of embodiments of the present invention more clearly, the accompanying drawings provided for describing the embodiments are introduced briefly in the following. The accompanying drawings in the following description are merely some embodiments of the present invention, modifications on these embodiments are possible without departing from the scope of the present invention as defined in the claims.
Figure 1 shows a schematic flowchart of a method for assisting multidisciplinary decision-making for treatment recommendations, according to one embodiment of the disclosure.
Figure 2 shows a schematic flowchart of an overall process embedding one embodiment of the disclosure.
Figures 3A and 3B show diagrams reflecting high-level and low-level treatment recommendations, including performance metrics and visualizations of individual patient recommendations and overall patient recommendations, which can be generated in one or more embodiments of the present disclosure.
Figure 4 shows an embodiment of an interactive dashboard displaying high-level and low-level treatment probabilities for a patient, according to one embodiment of the disclosure.

### DETAILED DESCRIPTION

In the following description, reference is made to drawings which show by way of illustration various embodiments. Also, various embodiments will be described below by referring to several examples. It is to be understood that the embodiments may include changes in design and structure without departing from the scope of the claimed subject matter.

The techniques described herein may be implemented in various computing systems, examples of which are described in greater detail below. Such systems generally involve the use of suitably-configured computing devices implementing a number of (hardware or software) modules, each providing one or more operations needed to complete execution of such techniques. Each module may be implemented in its own way; all need not be implemented the same way. As used herein, a module may be a structural component of a system which performs an operational role, which may be a portion of or an entire software element (e.g., a function of a process, a discrete process, or any other suitable embodiment). A module may comprise computer-executable instructions and may be encoded on a computer storage medium. Modules may be executed in parallel or serially, as appropriate, and may pass information between one another using a shared memory on the computer on which they are executing, using a message passing protocol or in any other suitable way. Exemplary modules are described below carrying out one or more tasks, though it should be appreciated that the modules and division of tasks described may be merely illustrative of the type of modules that may implement the exemplary techniques described herein, and that the invention is not limited to being implemented in any specific number, division, or type of modules. In some implementations, all functionalities may be implemented in a single module. Further, the modules may be discussed below as all executing on a single computing device for clarity, though it should be appreciated that, in some implementations, the modules may be implemented on separate computing devices adapted to communicate with one another.

The present disclosure may use various abbreviations. For example, AI or ML may be used to abbreviate the term Artificial Intelligence or Machine Learning, respectively. AI and ML may refers to the simulation of intelligence processes by computer systems. AI may be used as a synonym for ML. AI or ML systems according to the present disclosure are designed to perform tasks that typically require (human) intelligence, such as reasoning, problem-solving, learning, perception, and language understanding.

The term "trained model", as used throughout this disclosure, refers to an AI or ML model, which may be a machine learning tool configured to analyze input data and generate predictions, classifications, or decisions based on learned (or trained) patterns and relationships; it is set up by defining an architecture, selecting algorithms, and using training data to optimize its parameters, enabling it to process new input data and perform tasks such as identifying treatment categories or predicting specific treatment options. A trained model may be developed through a unsupervised or supervised learning process, wherein the trained model may be trained on (labeled) datasets by iteratively adjusting its parameters to learn patterns and relationships within the data, enabling it to perform specific tasks such as classifying patient features, identifying treatment categories, or predicting treatment options, based on (previously unseen) input patient data. The trained model may be implemented as software or hardware, or a combination of both, such as software implemented on AI accelerator hardware or graphics hardware.

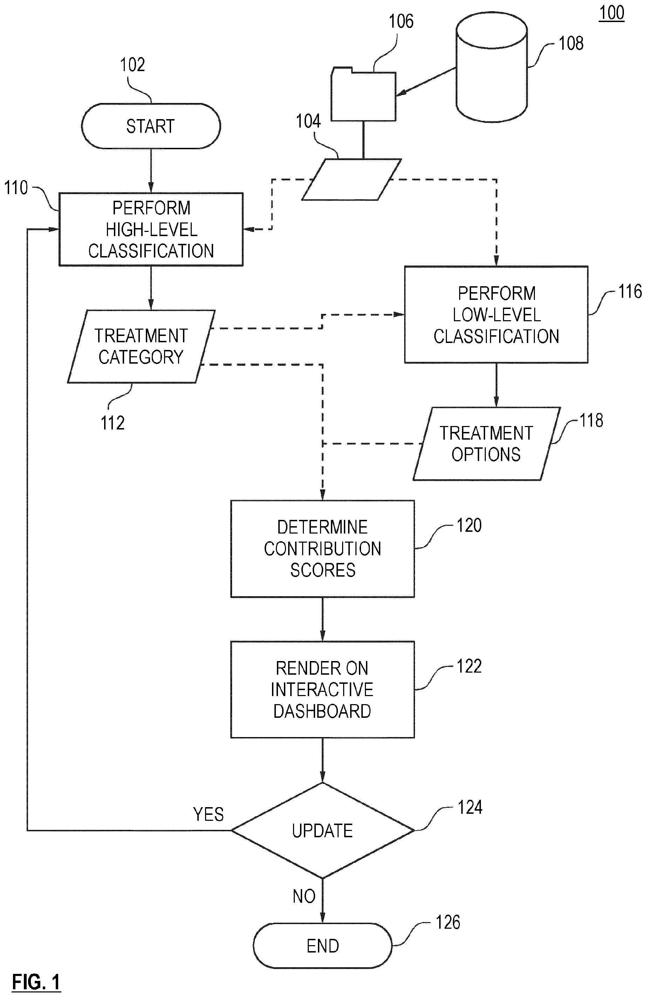

Figure 1 shows a schematic flowchart of a method for assisting multidisciplinary decision-making for treatment recommendations, according to one embodiment of the disclosure.

The method 100 begins at the start 102. The method 100 may use a two-tier inference system with a first-tier trained model and at least one second-tier trained model. The method 100 may access patient data, by accessing patient features 104 derived from a clinical record 106 of a patient, which may be stored in a database 108.

The method may perform a high-level classification 110 using a first-tier trained model. Step utilizes the patient features 104 derived from the clinical record 106 to identify a treatment category 112. The treatment category 112 may be used as a parameter to specify a model for a low-level classification in step 116.

At step 116, a low-level classification of the patient data 104 may be performed using at least one second-tier trained model. Step 116 may refine the treatment category 112 into at least one treatment options 118. For example, each second-tier trained model may be used to predict a treatment option of the at least one treatment options 118.

Subsequently, the method 100 may determine 120 contribution scores for at least some of the patient features 104. Each contribution score may indicate an impact of a corresponding patient feature on the identified treatment category 112 or the identified treatment options 118.

The method 100 may proceed with rendering 122 the identified treatment category 112, the identified treatment options 118, and one or more indications of the contribution scores on an interactive dashboard. The dashboard may provide a multi-modal, for example, a visual representation of the treatment recommendations and the underlying data.

The method 100 may then check if the patient record 106 has been update in item 124. In case of an update, the process may loop back to step 110 to incorporate new data or refine the existing recommendations. If no update is required, the process ends at block 126. However, it is to be understood that item 124 is optional.

Throughout the flowchart, the dashed lines represent the flow of data as input to the individual method steps, and further represent the interactions between different steps, ensuring that the method dynamically adapts to the patient data.

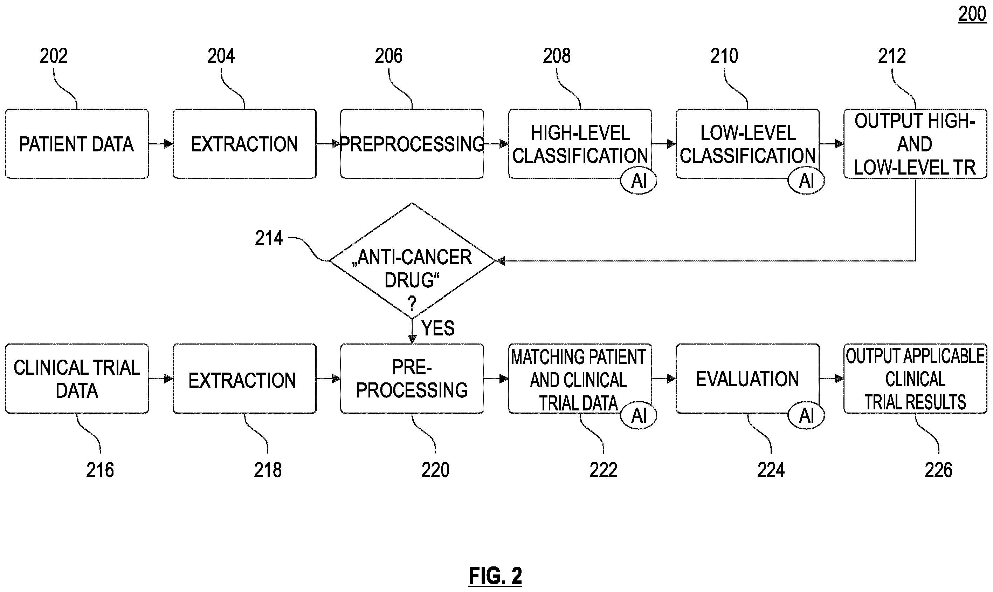

Figure 2 shows a schematic flowchart of an overall process embedding one embodiment of the disclosure.

The process 200 may begin with the acquisition of patient data 202, which may be subjected to extraction 204. Step 204 may include automated or semi-automated extraction. Step 204 may involve an identification and extraction of relevant features from raw patient data, ensuring that the data is in a suitable format for subsequent processing.

Following extraction in item 204, the data undergoes preprocessing 206. This step may include normalizing and standardizing the data, encoding categorical variables, and parsing complex data types into structured features. Preprocessing 206 may ensure that the data is in a consistent format, to enable analysis by subsequent classification models.

The preprocessed data may then be fed into a high-level classification model 208. This model 208 may utilize AI approaches to perform a broad classification of the patient data into general treatment categories. The high-level classification may serve as a preliminary sorting mechanism, directing the data towards the appropriate treatment category. Model 208 may, for example, be the first-tier trained model as discussed in step 110 of the method 100 in Figure 1.

Once the high-level classification 208 is complete, the data may proceed to a low-level classification model 210. This model 210, also powered by AI or ML technology, may refine the treatment category into specific treatment options. The low-level classification may take into account the (patient) features, which may correspond to the results of step 206. Moreover, the identified treatment category can be used to decide which low-level classification model should be used. The low-level classification 210 can be used to provide a more precise treatment recommendation. Model 210 may, for example, include the at least one second-tier trained model as discussed in step 116 of the method 100 in Figure 1.

The results of both the high-level and low-level classifications 208, 210 may then be output in step 212. This output may include the identified treatment category and the specific treatment options, which may be, for example, rendered on an interactive dashboard for further analysis and decision-making.

In parallel, clinical trial data 216 may be processed through a similar sequence of steps. Extraction 218 may be performed on the clinical trial data 216 to extract relevant clinical trial information. This may include automatic or semi-automatic extraction.

Based on the output 212, a determination can be made in step 214, whether the output includes a specific treatment option, such as an "anti-cancer drug" recommendation. In this case, the patient data resulting from step 206 and the low-level treatment option provided in step 210, such as an anti-cancer medication, can be processed with the extracted data 218 in step 220. Hence, extraction 218 may be followed by preprocessing 220, which may ensure that the clinical trial data is in a suitable format for comparison with the patient data (from step 206).

The preprocessed study data is then used in a matching process 222, where patient features of the patient data 206 are compared against clinical trial data. Moreover, the low level results from step 210 may be used in step 222. This matching process 222 may employ another trained model, such as a large language model. The matching may identify relevant clinical trial records that correspond to the identified treatment category and options.

An evaluation step 224 follows, where the matched clinical trial data is assessed to ensure its applicability to the patient's specific context. The evaluation process ensures that the clinical trial data is relevant and can be used to inform treatment recommendations.

The applicable clinical trial results may be provided in step 226. These results may be rendered alongside the patient data on the interactive dashboard, providing healthcare professionals with a comprehensive view of the treatment options and supporting clinical evidence.

Overall, Figure 2 provides a detailed depiction of a method for assisting multidisciplinary decision-making, highlighting the integration of patient data, study data, and AI-driven classification models to deliver precise and evidence-based treatment recommendations.

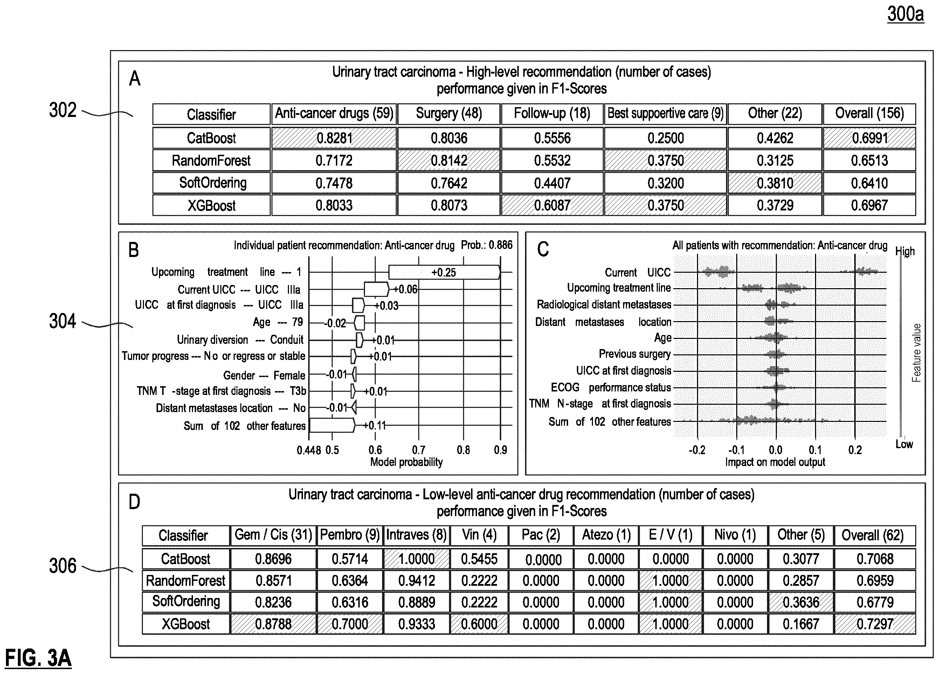

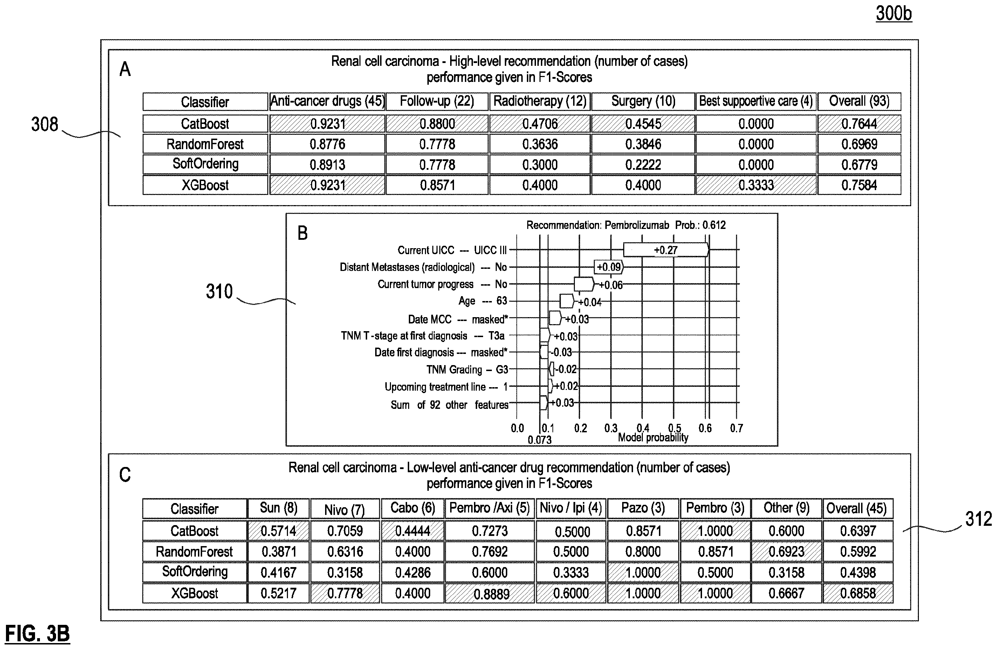

Figures 3A and 3B show diagrams reflecting high-level and low-level treatment recommendations, including performance metrics and visualizations of individual patient recommendations and overall patient recommendations, which can be generated in one or more embodiments of the present disclosure.

Figure 3A provides a visualization 300a of results of a two-tier prediction system that may be used to assist multidisciplinary decision-making for treatment recommendations based on patient data and clinical evidence. The results may be provided in multiple sections, each providing specific insights and data visualizations.

Section A of the visualization 300a, labeled with reference numeral 302, presents high-level recommendations categorized by the number of cases. The classifiers used in this section include CatBoost, Random Forest, SelfOrganizing, and XGBoost. The performance of these classifiers is given in Fl-Scores across various treatment categories such as Anti-cancer drugs, Surgery, Follow-up, Best supportive care, Other, and Overall. The table in this section provides a comparative analysis of the performance of each classifier in recommending the appropriate high-level treatment category.

Section B, labeled with reference numeral 304, focuses on individual patient recommendations for the option "anti-cancer drug". This section includes a bar chart that visualizes the probability of recommending anti-cancer drugs for individual patients based on various features. The features considered include the current UICC (Union for International Cancer Control) stage, age, comorbidities, tumor progression, and other clinical and demographic data. The chart highlights the impact of each feature on the model's probability of recommending anti-cancer drugs.

Section C, also part of reference numeral 304, provides a scatter plot that visualizes the impact of various features on the model output for all patients with recommendations for anti-cancer drugs. The x-axis represents the impact on the model output, while the y-axis lists the features such as upcoming treatment line, radiological distant metastases, previous surgery, ECOG (Eastern Cooperative Oncology Group) performance status, and other relevant clinical factors. This visualization helps in understanding the contribution of each feature to the model's decision-making process.

Section D, labeled with reference numeral 306, presents low-level anti-cancer drug recommendations categorized by the number of cases. Similar to Section A, this section includes a table that compares the performance of different classifiers (CatBoost, Random Forest, SelfOrganizing, and XGBoost) in recommending specific anti-cancer drugs. The performance is again given in F1-Scores across various drug categories such as Gem/Cis (Gemcitabine/Cisplatin), Pembro (Pembrolizumab), Atezo (Atezolizumab), E/V (Enfortumab-Vedotin) and other anti-cancer drugs. This section provides a detailed analysis of the classifiers' ability to recommend the most suitable anti-cancer drugs based on patient features.

Overall, Figure 3A illustrates high-level and low-level treatment recommendations. The visualization 300a leverages multiple classifiers to analyze patient data and clinical evidence that facilitates informed decision-making by healthcare professionals. The visualizations and performance metrics help in understanding the effectiveness of different classifiers and the impact of various patient features on treatment recommendations.

Even though Figure 3A shows a particular layout, this is intended to illustrate the various recommendations generated based on patient data and clinical data using embodiments of the present disclosure. Figure 3A is not intended to limit the present disclosure by a particular layout of a user interface.

Figure 3B illustrates a breakdown of the system's recommendation process for treatment options based on patient data and clinical evidence. The figure is divided into three main sections labeled A, B, and C, each representing different stages and aspects of the recommendation process.

Section A of the visualization 300b, labeled as 308, presents the high-level recommendation for treatment categories. This section includes a table that lists various classifiers such as CatBoost, Random Forest, XGBoost, and Soft-Ordering. Each classifier is evaluated based on its performance in recommending treatment categories, including Anti-cancer drugs, Follow-up care, Surgery, Radiotherapy, and Best supportive care. The table includes columns for each treatment category and an overall performance score, quantified using F1-scores. These scores indicate the effectiveness of each classifier in identifying the appropriate treatment category for a number of cases.

Section B, labeled as 310, provides a visual representation of the recommendation probabilities for a specific anti-cancer drug, designated as "Pembrolizumab". This section includes a chart that displays the model probability for Pembrolizumab, indicating the likelihood of this drug being recommended based on the patient features. The chart includes annotations and markers that highlight the contribution of various patient features to the recommendation probability, providing a visualization of the factors influencing the decision-making process.

Section C, labeled as 312, details the low-level recommendation for specific anti-cancer drugs within the identified treatment category. Similar to Section A, this section includes a table that lists the same classifiers and evaluates their performance in recommending specific anti-cancer drugs such as Sun, Nivo, Cabo, Pembro/Axi, Nivo/Ipi, Pazo, Pembro, and others. The table includes columns for each drug and an overall performance score, again quantified using F1-scores. These scores reflect the classifiers' effectiveness in identifying the most suitable anti-cancer drug for a number of cases.

Figure 3B as a whole demonstrates the system's capability to provide both high-level and low-level treatment recommendations. The high-level recommendations identify broad treatment categories, while the low-level recommendations refine these categories into specific treatment options. The inclusion of performance metrics for various classifiers and the visual representation of recommendation probabilities ensures transparency and explainability in the decision-making process. This systematic approach allows for a comprehensive evaluation of treatment options, enabling informed decision-making in a clinical setting.

Even though Figure 3B shows a particular layout, this is intended to illustrate the various recommendations generated based on patient data and clinical data using embodiments of the present disclosure. Figure 3B is not intended to limit the present disclosure by a particular layout of a user interface.

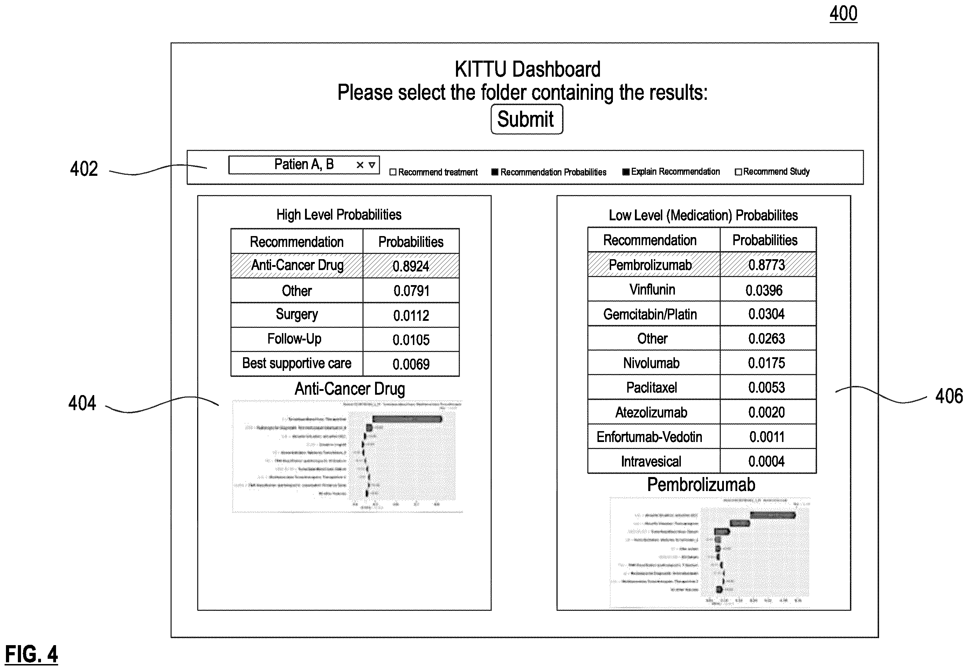

Figure 4 shows an embodiment of an interactive dashboard displaying high-level and low-level treatment probabilities for a patient, according to one embodiment of the disclosure. The dashboard 400, labeled as KITTU Dashboard, is segmented into two primary sections: High-Level Probabilities 404 and Low-Level (Medication) Probabilities 406.

The High-Level Probabilities section 404 displays a list of treatment categories along with their associated probabilities. These categories include Anti-Cancer Drug, Other, Surgery, Follow-Up, and Best Supportive Care. Each category is accompanied by a probability value, indicating the likelihood of that treatment being recommended for the patient. For instance, the Anti-Cancer Drug category has a probability of 0.8924, which is the highest among the listed categories, suggesting it is the recommended treatment. In case there are multiple accepted recommendations all of them could be highlighted in a similar manner. The probabilities difference between valid recommendations may highlight their representation in the training cohort and does not provide information about which of them is superior. Below this list, there is a detailed graphical representation of the Anti-Cancer Drug category, providing further insights into the factors contributing to this recommendation.

In the shown example, the Low-Level (Medication) Probabilities section 406 provides a more granular view of specific medication options within the identified treatment category. This section lists various medications along with their respective probabilities. For example, Pembrolizumab has a probability of 0.8773, making it the most accepted medication to be recommended within the Anti-Cancer Drug category. Other medications listed include Vinflunine, Gemcitabine/Platin, Nivolumab, Paclitaxel, Atezolizumab, Enfortumab-Vedotin, and Intravesical, each with their corresponding probabilities. Similar to the High-Level Probabilities section, there is a detailed graphical representation for Pembrolizumab, illustrating the factors influencing its recommendation.

At the top of the dashboard, there is a patient selection input field 402, allowing users to select the patient for whom the treatment recommendations are being generated. Additionally, there are options for submitting the selected patient data and toggling different types of recommendations, such as Recommended Treatment, Recommendation Probabilities, Explain Recommendation, and Recommend Study.

The interactive dashboard 400 is designed to provide a comprehensive and detailed view of treatment recommendations, generated using a two-tier inference system, according to one or more embodiments of the present disclosure. The high-level classification identifies broad treatment categories, while the low-level classification refines these categories into specific treatment options. The dashboard also includes visual aids, such as graphs and charts, to enhance the interpretability of the recommendations and the contribution scores of various patient features.

Overall, Figure 4 demonstrates the functionality and layout of the KITTU Dashboard, highlighting its role in enabling informed decision-making in a clinical setting by presenting treatment recommendations in an organized and interactive manner.

It is to be understood that the implementational details as provided in Fig. 1 to 4 represent preferred examples. Other implementations using different components, modules, blocks, units, circuitry, connections, and links can be used, and the present disclosure is not restricted by a particular implementation in silicon.

While some embodiments have been described in detail, it is to be understood that aspects of the disclosure can take many forms. In particular, the claimed subject matter may be practiced or implemented differently from the examples described, and the described features and characteristics may be practiced or implemented in any combination. The embodiments shown herein are intended to illustrate rather than to limit the invention as defined by the claims.

## Claims

1. A method for assisting multidisciplinary decision-making for treatment recommendations, comprising:
providing a two-tier inference system, including a first-tier trained model and at least one second-tier trained model;
accessing patient features derived from a clinical record of a patient;
performing a high-level classification with the first-tier trained model using the patient features to identify a treatment category for the patient;
performing a low-level classification with the at least one second-tier trained model using the patient features and the identified treatment category to identify one or more treatment options for the patient within the identified treatment category;
determining for at least some of the patient features contribution scores, each contribution score indicating an impact of a corresponding patient feature on the identified treatment category or the identified one or more treatment options; and
rendering at least one of the identified treatment category, the identified one or more treatment options, and one or more indications of the contribution scores on an interactive dashboard.

2. The method of claim 1, wherein the contribution scores are determined using an explanation engine configured to provide contribution scores for the patient and global contribution scores across patient cohorts.

3. The method of claim 1 or 2, further comprising generating labeled training data, and training the first-tier trained model and the at least one second-tier trained model using the labeled training data.

4. The method according to any one of the preceding claims, further comprising identifying datasets in the labeled training data with multiple labels and duplicating the identified datasets, wherein each duplicated dataset includes at least one different label of the multiple labels.

5. The method according to any one of the preceding claims, further comprising:
matching the patient features to records in a database with clinical trial data using a third trained model, thereby identifying one or more records specifying treatment characteristics for the identified treatment category and the one or more treatment options; and
rendering the treatment characteristics on the interactive dashboard.

6. The method according to claim 5, wherein matching the patient features to records in the database comprises:
parsing, using the third trained model, the records including unstructured clinical trial data into structured trial criteria;
comparing the patient features against the structured trial criteria; and
identifying the treatment characteristics in the structured trial criteria matching the patient features.

7. The method according to any one of the preceding claims, wherein the high-level classification identifies the treatment categories, including at least one of surgery, anti-cancer drugs, follow-up care, and best supportive care, and wherein the low-level classification identifies the one or more treatment options, including at least one of types of surgical procedures, at least one anti-cancer drug, at least one care procedure.

8. The method according to any one of the preceding claims, further comprising extracting patient features from the clinical records and preprocessing the patient features, including one or more of:
normalizing and standardizing numerical data,
encoding categorical variables, and
parsing complex data types into structured features.

9. The method according to any one of the preceding claims, wherein the patient features include one or more of demographic data, clinical data, biomarker data, laboratory results, histopathological findings, symptomatology, pathological data, imaging data, and parsed complex data types.

10. The method according to any one of the preceding claims, wherein the first-tier trained model and the at least one second-tier trained model include one or more of neural networks, support vector machines or boosting algorithms, tree-based approaches or ensemble methods, preferably with each trained model trained on labeled datasets to perform supervised learning for high-level classification of treatment categories and low-level classification of treatment options, and wherein the third trained model is a large language model.

11. The method according to any one of the preceding claims, wherein the first-tier trained model uses supervised learning to perform high-level classification of patient features into treatment categories, and the at least one second-tier trained model utilizes context-aware supervised learning to refine the treatment category into one or more treatment options based on patient features.

12. The method according to any one of the preceding claims, wherein the first-tier trained model is trained on labeled data representing treatment categories, and operates on patient features, to determine a most appropriate treatment category, and/or wherein the at least one second-tier trained model is trained on labeled data specific to treatment categories, operates on patient features, and uses the identified treatment category as an input parameter to focus the low-level classification and refine the one or more treatment options.

13. The method according to any one of the preceding claims, wherein the interactive dashboard is provided as a graphical user interface (GUI), including one or more dynamic components for interactive visualizations, wherein the interactive dashboard preferably provides evidence-based annotations linking treatment options to clinical trial data or medical guidelines, and interactive links to a source of clinical trial data for validation.

14. The method according to any one of the preceding claims, wherein the method is applicable in medical fields requiring multidisciplinary decision-making for treatment recommendations based on patient data and clinical evidence, including oncology, cardiology, and neurology, specifically including urological, gastrointestinal, gynecological, or thoracic oncology.

15. A system for assisting multidisciplinary decision-making for treatment recommendations, wherein the system is configured to perform a method according to any one of the claims 1 to 14.
